# EUROPEAN PATENT APPLICATION

(11) **EP 1 112 745 A1**
(43) Date of publication of application: **04.07.2001**
(21) Application number: 00100017.3
(22) Date of filing: 03.01.2000
(51) Int. Cl.: A61K 35/78, A61P 17/06

(54) **Homeopathic composition**

(71) Applicant: Maestri, Irma, 38085 Pieve di Bono (TN) (IT)
(72) Inventor: Maestri, Irma, 38085 Pieve di Bono (TN) (IT)
(74) Representative: Monti, Umberto

(57) **Abstract**

Homeopathic composition for local application in the treatment of psoriasis comprises, by weight:
ginger from 33% to 40%
black pepper from 2% to 3%
garlic (in gloves) from 6% to 7%
tobacco leaves from 4% to 6%
excipient q.s.

## Description

The present invention relates to a homeophatic composition for the treatment of skin diseases, and in particular of psoriasis.

Several remedies have been suggested for the treatment of psoriasis, comprising drugs to be ingested as well as medicaments for local application.

Besides, there have been suggested herb extracts such as Melia azadirachta and/or Centratherum anthelminthicum, extracts of prunella and others.

The object of the present invention is to provide a remedy for the treatment of skin diseases that avoid the use of pharmaceuticals, and more particularly to realize a composition of homeopathic type for local (topic) application.

As it is known, the allophatic methods provide for administering to a patient substances capable of producing effects contrary to those caused by the disease being treated, whereas on the other hand homeophatic medicaments are based on the administration of minimum amounts of substances that, in a healthy person, would induce the same symptoms of the disease to be treated.

According to this invention, the above object is achieved by a composition as claimed in claim 1, while further advantageous characteristics are recited in the dependent claims.

This invention will now be disclosed with reference to preferred but non limiting embodiments .

The composition of the invention is preferably implemented as a poultice or an emplastrum.

The composition of the invention comprises, by weight:
ginger from 33% to 40%
black pepper from 2% to 3%
garlic (in cloves) from 6% to 7%
tobacco leaves from 4% to 6%
excipient, such as a vegetal oil, from 45% to 31%.

More particularly, the ginger is preferably used fresh, cut into slices and slightly withered or dried in a pan without any additional ingredients, and then ground together with the other components. When dried, the ginger percentage has to be proportionally modified.

The tobacco has to be fresh. In an alternative composition, it is foreseen a content of naphthalene between 10% and 13%. In this case the naphthalene is the only non-vegetal component of the composition.

Preferably the vegetal oil, which is the excipient of the composition, is coconut oil or olive-oil and its amount depends on the desired liquidness degree for the product.

After grinding the ingredients and adding the vegetal oil, the product is let to macerate at least for a week before being used.

The actually preferred employment modality is the following.

The product is smeared on the part struck by the disease and then is protected by a preferably transparent material to avoid the escape of medicament, and then a bandage is applied.

The application of the composition is kept for about 6-7 hours each day, so that it is preferable to apply the treatment during the night. After removing the bandage, the skin can be washed with conventional products.

According to an embodiment of the invention, adapted for the treatment of the slightest cases, the composition is filtered and on the part to be treated is applied the oleosus liquid containing the indicated substances in suspension. As an example, such an embodiment is adapted to be applied to the lips.

Already in the first week of treatment week a disease remission has been reported, in particular the itching will completely disappear, this being the most troublesome symptom of this disease, while the albugineous branny lesions will progressively disappear and the anagenesis begins.

The treatment duration depends on the disease degree (slight or serious, acute or remittent) and on the type of the disease. In most cases, two weeks of treatment are sufficient, possibly with a repetition for 2-3 days in case of slight recidivation which nevertheless should occur after several months.

Although the invention has been illustrated with reference to preferred compositions, it is generally susceptible of other formulations and modifications falling within the scope of the invention, as it shall be evident to the skilled of the art.

## Claims

1. Homeopathic composition for local application in the treatment of psoriasis, characterised in that it comprises, by weight:
ginger from 33% to 40%
black pepper from 2% to 3%
garlic (in cloves) from 6% to 7%
tobacco leaves from 4% to 6%
excipient from 45% to 31%

2. Homeopathic composition as claimed in claim 1, characterised in that said excipient is a vegetal oil.

3. Homeopathic composition as claimed in claim 2, characterised in that said excipient is chosen between coconut and olive oil.

4. Homeopathic composition as claimed in claims 1 to 3, characterised in that the ginger is fresh, cut into slices and slightly withered or dried without additional ingredients.

5. Homeopathic composition as claimed in the preceding claims, characterised in that all the ingredients are ground before being added together.

6. Homeopathic composition as claimed in the preceding claims, characterised in that it is an oily supension obtained by colation.

7. Homeopathic composition as claimed in the preceding claims, characterised in that it further comprises naphthalene from 10% to 13%.
